# FASCICULE DE BREVET EUROPEEN

(11) **EP 4 061 912 B1**
(45) Date de publication et mention de la délivrance du brevet: **06.03.2024**
(21) Numéro de dépôt: 19832721.5
(22) Date de dépôt: 19.11.2019
(51) Int. Cl.: C10L 3/08, C07C 1/12, C07C 9/04, C12P 5/02

(54) **INSTALLATION DE PRODUCTION DE MÉTHANE**
ANLAGE ZUR HERSTELLUNG VON METHAN
PLANT FOR PRODUCING METHANE

(43) Date de publication de la demande: 28.09.2022
(73) Titulaire: Arkolia Energies, 34130 Mudaison (FR)
(72) Inventeur: VIALA, Audrey, 34130 MUDAISON (FR); SCUDELLER, Mickaël, 34130 MUDAISON (FR); BONHOMME, Laurent, 34130 MUDAISON (FR); HATTOU, Stéphane, 34130 MUDAISON (FR)
(74) Mandataire: Cornuejols, Marine Sophie
(86) Numéro de dépôt international: PCT/FR2019/052746
(87) Numéro de publication internationale: WO 2021/099695

(56) Documents cités:
- WO-A1-2012/110257
- WO-A1-2014/207703
- DE-A1-102009 018 126
- DE-A1-102011 013 922
- DE-B- 1 110 147
- FR-A1- 3 019 410
- FR-A1- 3 081 471
- ELHAM AHMADI MOGHADDAM ET AL: "Exploring the potential for biomethane production by willow pyrolysis using life cycle assessment methodology", ENERGY, SUSTAINABILITY AND SOCIETY, vol. 9, no. 1, 22 février 2019 (2019-02-22), XP055717532, DOI: 10.1186/s13705-019-0189-0

## Description

### DOMAINE TECHNIQUE

La présente invention concerne une installation et un procédé de production de méthane. Elle s'applique, en particulier, à la production de méthane basée sur de l'énergie solaire.

### ETAT DE LA TECHNIQUE

Le Power-to-Gas (que l'on peut traduire en français par « Energie vers gaz ») est une solution apportée à la croissance des installations de production d'énergies renouvelables. En effet, les énergies renouvelables sont des énergies variables, par conséquent il peut exister des pics de surproduction d'électricité à des moments de faible consommation électrique en France. C'est dans cette configuration que le Power-to-Gas prend tout son sens en créant une solution de stockage. En effet, la transformation de l'électricité en gaz permet de valoriser une perte d'électricité en vecteur énergétique via l'hydrogène ou le méthane.

Ce modèle Power-to-gas est construit comme suit : l'électricité générée par les sources renouvelables est transformée en hydrogène via un électrolyseur. Le processus peut s'arrêter à cette production et avoir plusieurs utilisations : injection dans les réseaux, mobilité, compression et stockage. Cependant, l'hydrogène (sous sa forme de gaz H₂) ne constitue pas un vecteur énergétique facilement stockable et transférable.

Le document WO 2012/110257 A divulgue un système pour stocker de l'énergie sous forme de méthane comprenant au moins un dispositif pour générer de l'énergie électrique à partir d'une source d'énergie renouvelable et/ou non-renouvelable, au moins un dispositif pour produire de l'hydrogène et/ou de l'oxygène par l'électrolyse d'eau et/ou de saumure, et au moins un bioréacteur comprenant une cuve de réaction appropriée pour la croissance, la fermentation et/ou la culture de microorganismes méthanogènes.

### EXPOSE DE L'INVENTION

La présente invention vise à remédier à tout ou partie de ces inconvénients.

A cet effet, la présente invention vise une installation de production de méthane qui comporte :
- une source d'énergie électrique,
- un électrolyseur d'eau alimenté en énergie électrique par la source d'énergie électrique, adapté à produire de l'hydrogène sous forme de gaz,
- un dispositif de capture de dioxyde de carbone atmosphérique adapté à fournir du dioxyde de carbone et de l'eau,
- un réacteur de méthanation muni d'une entrée d'hydrogène de l'électrolyseur, et d'une entrée d'eau et de dioxyde de carbone du dispositif de capture de dioxyde de carbone atmosphérique et adapté à produire du méthane et
- des capteurs solaires et un moyen de transfert de chaleur depuis les capteurs solaires au dispositif de capture de dioxyde de carbone.

Grâce à ces dispositions, on réduit le besoin d'eau de l'installation puisqu'au moins une partie du besoin d'eau du réacteur de méthanation est fournie par le dispositif de capture de dioxyde de carbone atmosphérique.

Dans des modes de réalisation, l'installation comporte un moyen de collecte d'eau de ruissellement des capteurs solaires, l'électrolyseur comportant une entrée d'eau de ruissellement collectée sur les capteurs solaires.

Dans des modes de réalisation, la source d'énergie électrique comporte des panneaux photovoltaïques, l'installation comportant un moyen de collecte d'eau de ruissellement des panneaux photovoltaïques l'électrolyseur comportant une entrée d'eau de ruissellement collectée sur les panneaux photovoltaïques.

Dans des modes de réalisation, l'installation comporte une unité de déshydratation du méthane sortant du réacteur de méthanation, le réacteur de méthanation comportant une entrée d'eau provenant de l'unité de déshydratation.

Dans des modes de réalisation, l'installation comporte une unité de déshydratation du méthane sortant du réacteur de méthanation, adaptée à fournir de l'eau à électrolyser en entrée de l'électrolyseur.

Chacun de ces modes de réalisation permet une économie d'eau.

Dans des modes de réalisation, l'électrolyseur est adapté à électrolyser de l'eau issue du réacteur de méthanation.

Dans des modes de réalisation, l'électrolyseur est adapté à électrolyser de l'eau sortant du dispositif de capture de dioxyde de carbone atmosphérique.

Dans des modes de réalisation, l'installation comporte un moyen de transfert de chaleur adapté à transférer de la chaleur depuis l'électrolyseur au dispositif de capture de dioxyde de carbone.

Dans des modes de réalisation, l'installation comporte un moyen de transfert de chaleur adapté à transférer de la chaleur du réacteur de méthanation au dispositif de capture de dioxyde de carbone.

Dans des modes de réalisation, la source d'énergie électrique comporte des panneaux photovoltaïques munis d'un système de refroidissement, l'installation comportant un moyen de transfert de chaleur adapté à transférer de la chaleur des panneaux photovoltaïques au dispositif de capture de dioxyde de carbone.

Dans des modes de réalisation, l'installation comporte un compresseur pour comprimer le méthane produit par le réacteur de méthanation et l'injecter dans un réseau de transport ou de distribution de gaz, et un moyen de transfert de chaleur adapté à transférer de la chaleur du compresseur au dispositif de capture de dioxyde de carbone.

Dans des modes de réalisation, l'électrolyseur est adapté à fournir une partie de la chaleur nécessaire au fonctionnement du dispositif de capture de dioxyde de carbone.

Dans des modes de réalisation, le réacteur de méthanation est adapté à fournir une partie de la chaleur nécessaire au fonctionnement du dispositif de capture de dioxyde de carbone.

Dans des modes de réalisation, la source d'énergie électrique comporte des panneaux photovoltaïques munis d'un système de refroidissement adapté à fournir une partie de la chaleur nécessaire au fonctionnement du dispositif de capture de dioxyde de carbone.

Dans des modes de réalisation, l'installation comporte un compresseur pour comprimer le méthane produit par le réacteur de méthanation et l'injecter dans un réseau de transport ou de distribution de gaz, une partie de la chaleur nécessaire au fonctionnement du dispositif de capture de dioxyde de carbone étant fournie par le compresseur.

Chacun de ces modes de réalisation économise de la chaleur et évite d'avoir à consommer de l'énergie électrique produite par les panneaux photovoltaïques pour fournir de la chaleur au dispositif de capture de dioxyde de carbone.

Dans des modes de réalisation, le réacteur de méthanation est un réacteur de bio-méthanation.

Dans des modes de réalisation, le réacteur de méthanation est un réacteur de méthanation catalytique.

Dans des modes de réalisation, le réacteur de méthanation est un réacteur de bio-méthanation par voie thermochimique.

### BREVE DESCRIPTION DES FIGURES

D'autres avantages, buts et caractéristiques de la présente invention ressortiront de la description qui va suivre faite, dans un but explicatif et nullement limitatif, en regard des dessins annexés, dans lesquels :
- la figure 1 représente, sous forme d'un schéma bloc, un mode de réalisation particulier de l'installation de production d'énergie mixte objet de l'invention,
- la figure 2 représente, sous forme d'un schéma bloc, les transferts de chaleurs entre différentes parties de l'installation illustrée en figure 1,
- la figure 3 représente, sous forme d'un schéma bloc, en plus des transferts de chaleur, les transferts d'eau entre différentes parties de l'installation illustrée en figures 1 et 2 et
- la figure 4 représente, sous forme de courbes, le fonctionnement de différents systèmes de l'installation illustrée en figures 1 à 3.

### DESCRIPTION DE MODES DE REALISATION DE L'INVENTION

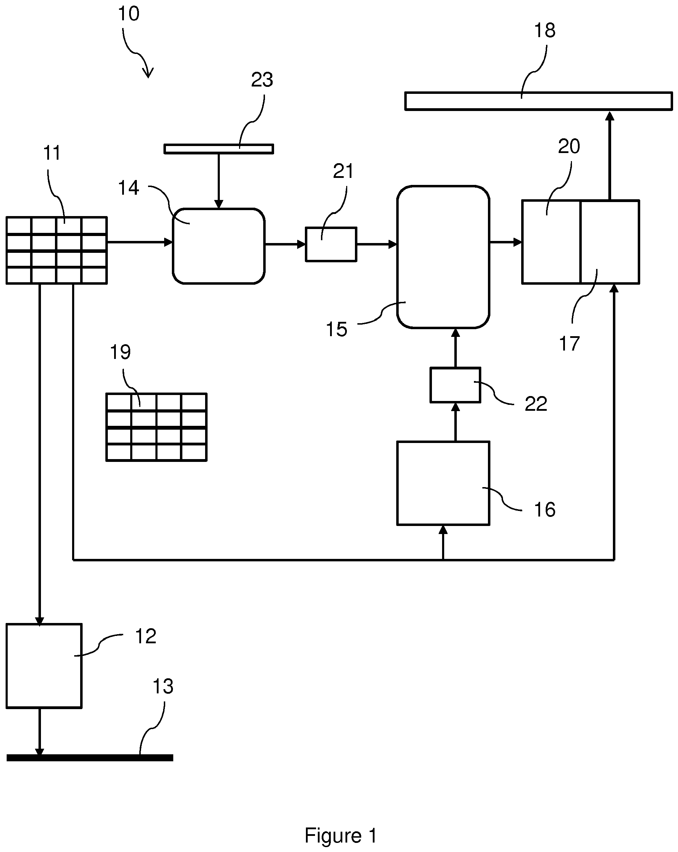

On note que les figures ne sont pas à l'échelle. On observe, en figure 1, une installation 10 de production d'énergie mixte, énergie sous forme d'électricité injectée dans un réseau électrique 13 et de méthane injecté dans un réseau de transport et/ou de distribution de gaz 18. L'installation 10 comporte une source d'énergie électrique, par exemple des panneaux photovoltaïques (ou « solaires »), produisant de l'électricité en courant continu et un transformateur 12 qui produit de l'électricité en courant alternatif, à la tension et à la fréquence du réseau électrique 13.

Dans des variantes, les panneaux photovoltaïques 11 sont remplacés par une autre source d'électricité dont la production peut être excédentaire par rapport à la consommation sur le réseau électrique ou par rapport à la capacité de transport de la partie du réseau électrique reliée à la source d'électricité, par exemple des éoliennes, voire une centrale nucléaire.

L'installation 10 comporte aussi, une unité d'électrolyse (ou « électrolyseur ») 14 produisant du dihydrogène (H₂) qui est stocké dans un réservoir 21. Le réservoir 21 est, par ailleurs, relié par l'intermédiaire d'un tuyau muni d'une vanne à une entrée d'un réacteur de méthanation 15. Une source 16 de dioxyde de carbone (CO₂) produit du dioxyde de carbone qui est stocké dans un réservoir 22. Le réservoir 22 est, par ailleurs, relié par l'intermédiaire d'un tuyau muni d'une vanne au réacteur de méthanation 15. Une unité de déshydratation 20 associé, en sortie, à un compresseur 17 fournissent du méthane sous pression au réseau de gaz 18. Selon des modes de réalisation, le réacteur 15 de méthanation est un réacteur de bio-méthanation, un réacteur de méthanation catalytique ou un réacteur de bio-méthanation par voie thermochimique.

Dans le cas d'un réacteur de bio-méthanation 15, des bactéries consomment le dihydrogène (H₂) et le dioxyde de carbone (CO₂) pour produire du méthane (CH₄) selon l'équation :

4H₂ + CO₂ → CH₄ + 2 H₂O

Comme l'indique cette équation, cette production de méthane s'accompagne d'un dégagement de chaleur et d'une production d'eau.

La méthanation biologique, ou bio-méthanation, est un procédé biologique, qui s'effectue à une température comprise entre 40 et 80 °C et à une pression inférieure à 10 bars. La bio-méthanation permet la conversion de dihydrogène (H₂) et de gaz carbonique (CO₂) en méthane (CH₄) au moyen d'un consortium microbien anaérobie. On distingue deux types de bio-méthanation :
- In situ : l'hydrogène est injecté dans un méthaniseur, à l'endroit où l'activité hydrogénotrophe est la plus favorable et
- Ex situ : l'hydrogène est injecté dans un réacteur dédié, dont le fonctionnement est réalisé dans des conditions optimisées (température, temps de séjour, typologie de biomasse et apport en nutriments).

Le rendement énergétique (calculé en kWhPCS) du réacteur de bio-méthanation est de 78 % pour la production de méthane (CH₄) et de 20 % en chaleur. Concernant les conversions d'H₂ et de CO₂ : au moins 98% de l'H₂ est convertie, et le rendement de conversion des atomes de carbone du gaz carbonique CO₂ en CH₄ est d'au moins 98%.

La bio-méthanation a comme avantage que son fonctionnement peut être intermittent : une fois les conditions de stabilité acquises, les bactéries sont peu sensibles aux variations de quantités de gaz au niveau de la charge d'alimentation en H₂ et CO₂ : cette charge peut devenir nulle ou revenir au débit nominal sans incidence sur la conversion de CO₂ et H₂ en CH₄. La bio-méthanation est ainsi adaptée aux photovoltaïque au sol. De plus, en ce qui concerne le dioxyde de carbone (CO₂), la méthanation présente peu de sensibilité aux imperfections et peut accepter des faibles teneurs en sulfure d'hydrogène (H₂S), en ammoniac (NH₃) et en dioxygène (O₂).

Dans des modes de réalisation moins préférentiels, on remplace la bio-méthanation par une méthanation catalytique de même équation chimique. Cette méthanation chimique s'effectue à une température comprise entre 250 et 350 °C, à une pression comprise entre 10 et 20 bars et est sensible au sulfure. Dans des modes de réalisation, on réalise la bio-méthanation par voie thermochimique.

La source 16 de dioxyde de carbone (CO₂) est préférentiellement un dispositif de capture du dioxyde de carbone par extraction atmosphérique. Une telle source est, par exemple, décrite dans le document US 2017 326 494, incorporé par référence, et dans les documents qui y sont cités, eux aussi incorporés par référence.

La séparation de gaz par adsorption permet de cibler un composant spécifique d'un courant gazeux. Une application importante consiste à capturer du dioxyde de carbone (CO₂) à partir de courants gazeux, par exemple, à partir d'air atmosphérique.

Plusieurs méthodes de capture de CO₂ atmosphérique ont été développées sur la base de diverses approches technologiques. Par exemple, le brevet US 8 163 066 décrit des structures et des techniques de capture et régénération de dioxyde de carbone. Le document US 2009/0120288 décrit un procédé pour éliminer le dioxyde de carbone de l'air. Le document US 2012/0174778 décrit un procédé de capture et régénération de dioxyde de carbone utilisant un ascenseur vertical. Le document WO2010022339 décrit un procédé et une installation de capture de dioxyde de carbone. Ces quatre documents sont incorporés ici par référence.

Une approche particulière est basée sur un processus d'adsorption / désorption cyclique sur des matériaux solides et chimiquement fonctionnalisés. Par exemple, dans le document WO2010 091831, une structure basée sur des matériaux adsorbants solides fonctionnalisés par une amine conjointement avec un procédé d'adsorption / désorption cyclique utilisant ce matériau pour l'extraction de dioxyde de carbone de l'air ambiant est décrite. Ce document est incorporé ici par référence.

Dans ce cas, le processus d'adsorption a lieu dans les conditions ambiantes où l'air est diffusé à travers le matériau adsorbant et une partie du CO₂ contenu dans l'air est chimiquement liée à la surface fonctionnalisée par l'amine du matériau. Pendant la désorption subséquente, le matériau est chauffé à environ 50-110 ° C et la pression partielle de dioxyde de carbone entourant le matériau est réduite en appliquant un vide ou en exposant le matériau à un écoulement de gaz de purge. Ainsi, le dioxyde de carbone capturé précédemment est éliminé du matériau adsorbant et obtenu sous une forme concentrée.

Dans le document WO2012 168346A1, incorporé ici par référence, on décrit un matériau à base de cellulose fonctionnalisée par une amine, qui peut être utilisé pour le procédé décrit ci-dessus.

Le matériau est de préférence un matériau modifié par amine, de préférence à base de résine échangeuse d'ions de base faible, en particulier de matériau de matrice de polystyrène modifié avec des groupes amine, spécialement des groupes amine primaire ou à base de cellulose, de la cellulose nanofibrilée, dans chaque cas de préférence avec un diamètre de particule moyen dans la plage de 60 à 1200 µm, pour l'adsorption de dioxyde de carbone.

Dans un procédé typique d'adsorption et désorption cyclique, l'adsorption peut avoir lieu aux conditions ambiantes, par exemple dans une plage de température de -30 à 40 ° C et à une pression absolue de 0,7 à 1,3 bar.

Après l'adsorption du dioxyde de carbone ou du dioxyde de carbone et de la vapeur d'eau, le matériau adsorbant peut être régénéré ou désorbé en le chauffant jusqu'à, par exemple 50 à 120 °C, et en réduisant la pression partielle de CO₂ autour du matériau adsorbant en réduisant la pression absolue à, par exemple, 1 à 250 mbar, à savoir, appliquer un vide et / ou exposer le matériau à un flux de gaz de purge. Si la désorption est obtenue en chauffant le matériau et en appliquant un vide, l'ensemble du processus cyclique est appelé processus de variation de température et de vide (dont l'acronyme est TVS). Si la désorption est réalisée en chauffant le matériau et en l'exposant à un flux de gaz de purge, le processus cyclique global est appelé processus de balayage de concentration en température (dont l'acronyme est TCS).

Dans des modes de réalisation, les couches de matériau individuelles sont formées sur des structures de châssis rigides, chacune comprenant un cadre rectangulaire avec une longueur de bord de 0,5 m × 0,6 m et une hauteur de 1 cm, constitué de profilés en acier inoxydable.

La fabrication et l'assemblage de la structure globale comprennent les étapes suivantes :
1. Les structures de châssis sont fabriquées par soudage de profilés en acier inoxydable ou par moulage par injection d'une matière plastique ou d'aluminium ;
2. Dans les cadres, un grillage en aluminium et un tube en caoutchouc contenant un fluide caloporteur sont insérés pour assurer un bon transfert de chaleur ;
3. Une couche de tissu non tissé est collée de chaque côté (haut et bas) de la structure du cadre en utilisant un adhésif à deux composants.
4. Un matériau à base de fibres de cellulose modifiées par une amine est produit selon une version agrandie de la procédure décrite dans le document WO2012 168346 :
   a. Isolement des nanofibres de cellulose à partir de la suspension raffinée de pâte de bois de hêtre fibreuse (voir "1. Isolement de nanofibres de cellulose" dans WO2012 168346 incorporé ici par référence),
   b. Ajout d'une solution de 3-aminopropylméthyldiéthoxysilane hydrolysée à une suspension de nanofibres ayant une teneur en masse sèche de 3,2%,
   c. Homogénéisation et agitation de la solution pendant deux heures,
   d. Congélation de la solution par lots dans du cuivre se fait dans l'azote liquide,
   e. Lyophilisation du mélange congelé pendant 48 heures,
   f.Traitement de la matière séchée à 120 °C dans un four sous atmosphère d'argon, et
   g. Compression et râpe du matériau pour obtenir un matériau granulaire avec une taille moyenne de particules d'environ 400 µm ;
5. Le matériau est injecté dans la structure du cadre à travers un trou qui est ensuite fermé ;
6. Une couche de matériau absorbant est obtenue. L'épaisseur de cette couche varie entre 1 et 1,5 cm ;
7. Au total, 26 cadres sont empilés les uns sur les autres tandis que des pièces distantes forment les canaux d'entrée et de sortie ;
8. L'empilement est monté à l'intérieur d'une chambre à vide rectangulaire ayant des dimensions internes de 0,55 m × 0,55 m × 0,65 m avec une entrée et une ouverture de sortie pour le flux d'air ;
9. Une vanne papillon actionnée pneumatiquement est connectée à chacune des ouvertures pour sceller et ouvrir la chambre vers l'environnement ;
10. L'ouverture d'entrée est connectée à un ventilateur pour produire le flux d'air pendant l'adsorption. En outre, la chambre est connectée à une pompe à vide pour réduire la pression pendant la désorption et à un thermostat pour chauffer et refroidir l'empilement pendant les étapes du cycle individuel.

Avec la structure proposée, l'extraction d'une partie substantielle du CO₂ contenu dans un flux d'air atmosphérique en utilisant un matériau modifié aux amines est possible à une chute de pression inférieure à 100 Pa avec une structure pouvant être fabriquée avec un effort limité.

Dans le cas, représenté en figure 1, où la source 16 de dioxyde de carbone est un dispositif de capture du dioxyde de carbone de l'air atmosphérique, des capteurs solaires 19 chauffent un fluide caloporteur (eau sous-pression, huile ou sels fondus).

Dans des modes de réalisation, le CO₂ provient d'un biogaz issu d'un méthaniseur traitant par exemple des boues de station d'épuration.

L'unité de déshydratation 20 permet au méthane injecté dans le réseau de gaz 18 de répondre aux spécifications du gaz fourni aux consommateurs reliés à ce réseau 18. On note que cette unité de déshydratation produit de l'eau. Le compresseur 17 comprime le méthane sous pression. Du fait des lois de la thermodynamique, le compresseur 17 produit aussi de la chaleur. De plus, les spécifications du réseau de gaz 18 peuvent imposer que le gaz comprimé soit refroidi, ce qui implique un quantité de chaleur supplémentaire à collecter.

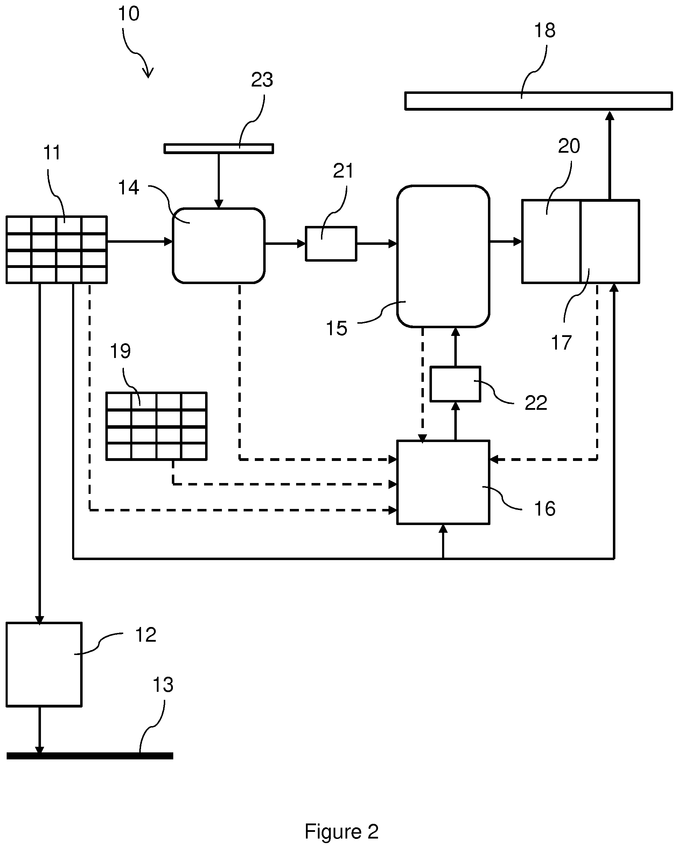

En figure 2, les transferts de chaleur sont représentés en traits discontinus fins. Ces transferts de chaleur sont réalisés par des moyens de transfert de chaleur (non représentés) comportant des échangeurs de chaleur et un moyen de déplacement de fluide caloporteur dans des tuyaux munis de vannes (non représentés) entre les échangeurs de chaleur (non représentés). Comme illustré en figure 2, dans le cas d'une source 16 de CO₂ nécessitant de la chaleur, par exemple un dispositif de capture du dioxyde de carbone atmosphérique, cette chaleur est, au moins partiellement, produite et fournie par :
- des capteurs solaires 19 chauffant un fluide caloporteur (par exemple eau sous-pression, huile ou sels fondus) ;
- l'unité d'électrolyse 14 (préférentiellement, 20 à 40 % des besoins de chaleur de la source 16) ;
- le réacteur de méthanation 15 (préférentiellement 20 à 40 % des besoins de chaleur de la source 16) ;
- un système de refroidissement (non représenté) des panneaux photovoltaïque 11 et/ou
- le compresseur 17.

Optionnellement, une unité de géothermie (non représentée) fournit de la chaleur à la source 16.

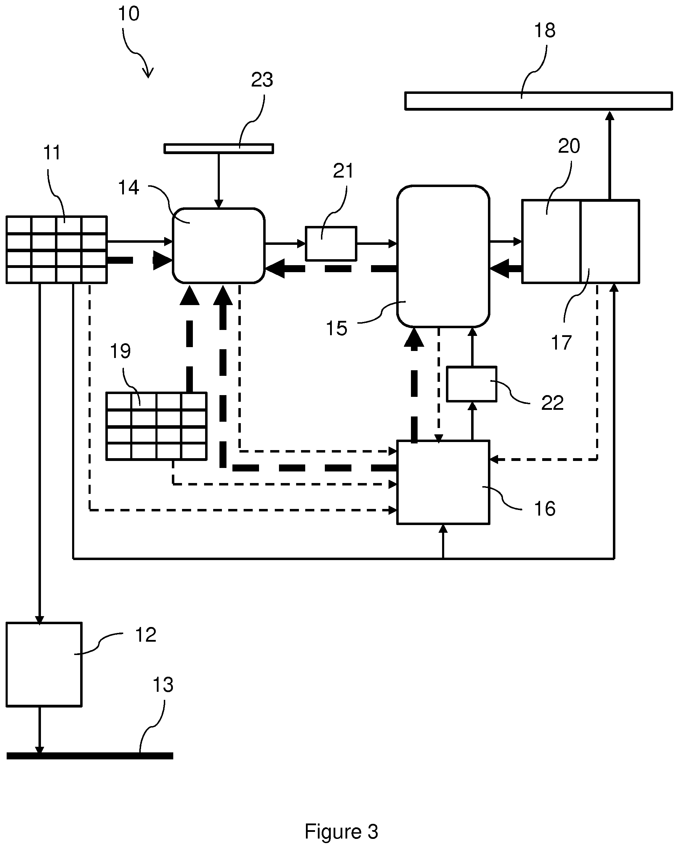

En figure 3, les transferts d'eau sont représentés en traits discontinus épais. Ces transferts d'eau correspondant à des tuyaux munis de vannes (non représentés). Comme illustré en figure 3, de l'eau produite par la source 16 de dioxyde de carbone (CO₂) est transférée au réacteur de méthanation 15 et utilisée dans le réacteur de méthanation 15, par exemple pour diluer les nutriments nécessaires au consortium microbien anaérobie. Préférentiellement, l'eau produite par la source 16 de dioxyde de carbone (CO₂) couvre l'intégralité des besoins d'eau du réacteur de méthanation 15 lors de son fonctionnement continu. En d'autres termes, le réacteur de méthanation ne comporte par d'autre entrée d'eau qui est ouverte lors de son fonctionnement continu, que celle recevant de l'eau produit par la sourcé 16 de dioxyde de carbone.

De l'eau produite par l'unité de déshydratation 20 est transportée en entrée du réacteur de méthanation 15 et/ou de l'unité d'électrolyse 14. De l'eau produite par le réacteur de méthanation 15 est transportée en entrée de l'unité d'électrolyse 14. Préférentiellement, entre 40 % et 80 % de l'eau utilisée par l'unité d'électrolyse 14 peut être fournie par le réacteur de méthanation 15. On note, néanmoins, qu'il est alors préférable de purifier l'eau. Par ailleurs, l'installation comporte un moyen (non représenté) de collecte d'eau de ruissellement des panneaux photovoltaïques 11 et/ou des capteurs solaires 19. Cette eau de ruissellement collectée est transportée en entrée de l'unité d'électrolyse 14 pour couvrir, par exemple, jusqu'à 40 % des besoins d'eau de l'unité d'électrolyse 14. Comme illustré en figure 3, optionnellement, une partie (par exemple, 15 à 35 %) de la consommation d'eau de l'unité d'électrolyse 14 est de l'eau produite par la source 16 de dioxyde de carbone (CO₂).

Dans des variantes (non représentées), l'intégralité de la production électrique provenant des panneaux photovoltaïques 11 est utilisée pour produire du méthane, et non uniquement l'excédent d'électricité.

Comme on le comprend à la lecture de la description qui précède, l'installation 10 de production de méthane comporte notamment :
- une source d'énergie électrique 11,
- un électrolyseur d'eau 14 alimenté en énergie électrique par la source d'énergie électrique, adapté à produire de l'hydrogène sous forme de gaz,
- un dispositif 16 de capture de dioxyde de carbone atmosphérique adapté à produire du dioxyde de carbone et de l'eau,
- un réacteur 15 de méthanation muni d'une entrée d'hydrogène de l'électrolyseur, et d'une entrée d'eau et de dioxyde de carbone du dispositif de capture de dioxyde de carbone atmosphérique et adapté à produire du méthane et
- des capteurs solaires 19 et un moyen de transfert de chaleur depuis les capteurs solaires au dispositif de capture de dioxyde de carbone.

L'installation 10 présente de nombreux avantages. Les centrales d'énergie renouvelable produisent une électricité intermittente à prix bas. Une partie excédentaire de l'électricité produite peut être utilisée, via une électrolyse de l'eau, à produire de l'H₂ qui est stockable mais dans des conditions assez coûteuses. Le captage et le stockage de CO₂ est une solution d'avenir pour réduire la pollution des industries et des villes mais aussi répondre à l'engagement de diviser par quatre les émissions de CO₂ d'ici 2050. L'installation combine les avantages de la capture et du stockage du CO₂ avec ceux de l'électrolyse de l'eau en mettant en oeuvre une méthanation et des synergies en termes de flux de chaleur et de flux d'eau. On obtient ainsi non seulement le captage du CO₂ de l'air, mais aussi la valorisation du CO₂ en énergie, une étape de plus dans la lutte contre le réchauffement climatique mais également pour l'indépendance énergétique. De l'eau étant produite par cette technologie de méthanation (pour la bio-méthanation, sensiblement une tonne d'eau par tonne de dioxyde de carbone capté), on réduit le besoin en eau de l'installation de production d'énergie mixte.

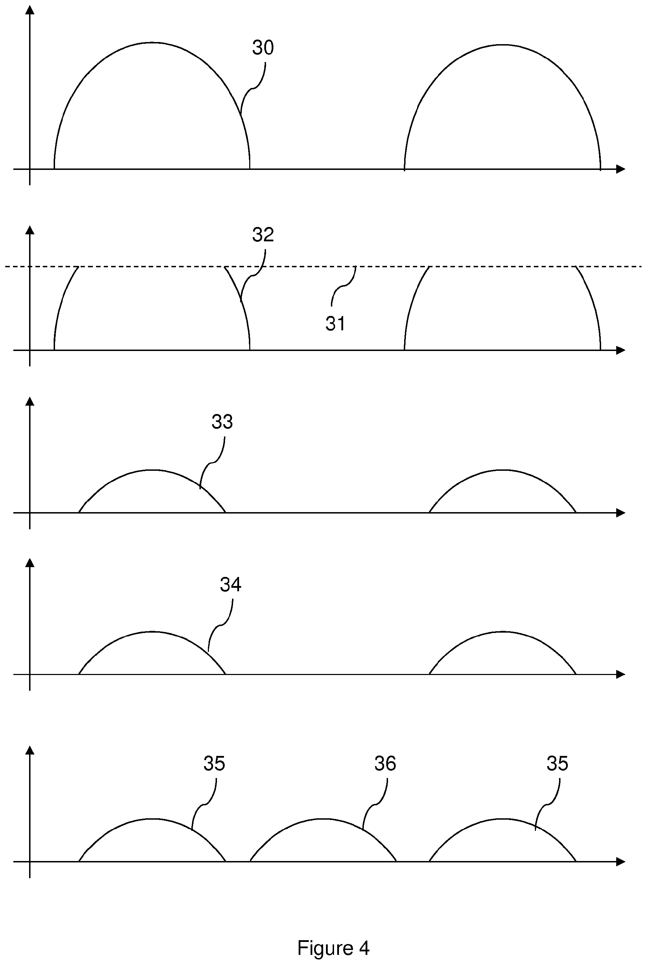

On observe, en figure 4, une courbe 30 de production d'électricité au cours du temps pendant deux journées successives. Cette courbe présente un lobe pour chaque journée d'ensoleillement, la production nocturne étant nulle.

On observe aussi une valeur limite 31 de production d'électricité au-delà de laquelle la production d'électricité est excédentaire, le réseau électrique 13 ne pouvant pas absorber l'électricité produite au-delà de cette valeur limite 31. Cette valeur limite 31 peut être fixe dans le temps, par exemple à cause du dimensionnement des câbles électriques reliés à l'installation 10, comme on le suppose en figure 4. Cette valeur limite 31 peut aussi être variable, en fonction de la consommation d'énergie sur le réseau électrique 13. Seule la partie 32 de l'électricité produite est alors fournie au réseau électrique 13. La partie 33 de l'électricité produite alimente les systèmes de l'installation 10, notamment l'unité d'électrolyse 14. La quantité de dihydrogène produite est représentée par la courbe 34. Les courbes 35 et 36 représentent le fonctionnement de la source 16 de dioxyde de carbone.

Préférentiellement, comme illustré en figure 4, pendant les heures nocturnes froides, la source 16 adsorbe le dioxyde de carbone (CO₂) atmosphérique, selon la courbe 36. Au contraire, pendant les heures diurnes les plus chaudes, la source 16 libère le dioxyde de carbone adsorbé et reçoit de la chaleur des différents composants de l'installation, comme exposé en regard de la figure 3. En variante, la capture et la fourniture de dioxyde de carbone (CO₂) par la source 16 sont réalisées alternativement de manière continue tout au long de la période de fourniture d'électricité par les panneaux photovoltaïques 11.

En variante (non représentée), l'intégralité de l'électricité produite par les panneaux photovoltaïque est utilisée pour générer du méthane. En d'autres termes, la valeur limite 31 est nulle.

En variante (non représentée), selon les saisons, on produit plus ou moins d'électricité ou de méthane, en faisant varier la valeur limite 31. Par exemple, en hiver, période de l'année où la consommation de gaz (pour le chauffage) est maximale, l'installation 10 est essentiellement utilisée pour produire du méthane, la valeur limite 31 étant alors faible ou nulle. En revanche, en été, période où la consommation de gaz est minimale, l'installation 10 est essentiellement utilisée pour produire de l'électricité, la valeur limite 31 étant alors plus élevée.

## Revendications

1. Installation (10) de production de méthane, **caractérisée en ce qu'**elle comporte :
- une source d'énergie électrique (11),
- un électrolyseur d'eau (14) alimenté en énergie électrique par la source d'énergie électrique, adapté à produire de l'hydrogène sous forme de gaz,
- un dispositif (16) de capture de dioxyde de carbone atmosphérique adapté à produire du dioxyde de carbone et de l'eau,
- un réacteur (15) de méthanation muni d'une entrée d'hydrogène de l'électrolyseur, et d'une entrée d'eau et de dioxyde de carbone du dispositif de capture de dioxyde de carbone atmosphérique et adapté à produire du méthane et
- des capteurs solaires (19) et un moyen de transfert de chaleur depuis les capteurs solaires au dispositif (16) de capture de dioxyde de carbone.

2. Installation (10) selon la revendication 1, qui comporte un moyen de collecte d'eau de ruissellement des capteurs solaires (19), l'électrolyseur (14) comportant une entrée d'eau de ruissellement collectée sur les capteurs solaires (19).

3. Installation (10) selon l'une des revendications 1 ou 2, dans laquelle la source d'énergie électrique (11) comporte des panneaux photovoltaïques, l'installation comportant un moyen de collecte d'eau de ruissellement des panneaux photovoltaïques (11) l'électrolyseur (14) comportant une entrée d'eau de ruissellement collectée sur les panneaux photovoltaïques (11).

4. Installation (10) selon l'une des revendications 1 à 3, qui comporte une unité (20) de déshydratation du méthane sortant du réacteur de méthanation (15), le réacteur de méthanation comportant une entrée d'eau provenant de l'unité de déshydratation.

5. Installation (10) selon l'une des revendications 1 à 4, qui comporte une unité (20) de déshydratation du méthane sortant du réacteur de méthanation (15), adaptée à fournir de l'eau à électrolyser en entrée de l'électrolyseur (14).

6. Installation (10) selon l'une des revendications 1 à 5, dans laquelle l'électrolyseur (14) est adapté à électrolyser de l'eau issue du réacteur de méthanation (15).

7. Installation (10) selon l'une des revendications 1 à 6, dans laquelle l'électrolyseur (14) est adapté à électrolyser de l'eau sortant du dispositif (16) de capture de dioxyde de carbone atmosphérique.

8. Installation (10) selon l'une des revendications 1 à 7, qui comporte un moyen de transfert de chaleur adapté à transférer de la chaleur depuis l'électrolyseur (14) au dispositif (16) de capture de dioxyde de carbone.

9. Installation (10) selon l'une des revendications 1 à 8, qui comporte un moyen de transfert de chaleur adapté à transférer de la chaleur du réacteur de méthanation (15) au dispositif (16) de capture de dioxyde de carbone.

10. Installation (10) selon l'une des revendications 1 à 9, dans laquelle la source d'énergie électrique comporte des panneaux photovoltaïques (11) munis d'un système de refroidissement, l'installation comportant un moyen de transfert de chaleur adapté à transférer de la chaleur des panneaux photovoltaïques au dispositif (16) de capture de dioxyde de carbone.

11. Installation (10) selon l'une des revendications 1 à 10, qui comporte un compresseur (17) pour comprimer le méthane produit par le réacteur de méthanation (15) et l'injecter dans un réseau (18) de transport ou de distribution de gaz, et un moyen de transfert de chaleur adapté à transférer de la chaleur du compresseur au dispositif (16) de capture de dioxyde de carbone.

12. Installation (10) selon l'une des revendications 1 à 11, dans laquelle le réacteur (15) de méthanation est un réacteur de bio-méthanation.

13. Installation (10) selon l'une des revendications 1 à 11, dans laquelle le réacteur (15) de méthanation est un réacteur de méthanation catalytique.

14. Installation (10) selon l'une des revendications 1 à 11, dans laquelle le réacteur (15) de méthanation est un réacteur de bio-méthanation par voie thermochimique.

## Patentansprüche

1. Anlage (10) zur Herstellung von Methan, **dadurch gekennzeichnet, dass** sie umfasst:
- eine elektrische Energiequelle (11);
- einen mit elektrischer Energie aus der elektrischen Energiequelle versorgten Wasserelektrolyseur (14), der zum Erzeugen von Wasserstoff in Gasform geeignet ist;
- eine Kohlendioxid-Abscheidungsvorrichtung aus der Atmosphäre (16), die zum Erzeugen von Kohlendioxid und Wasser geeignet ist;
- einen Methanisierungsreaktor (15), der mit einem Einlass für Wasserstoff aus dem Elektrolyseur und mit einem Einlass für Wasser und Kohlendioxid aus der Kohlendioxid-Abscheidungsvorrichtung aus der Atmosphäre ausgestattet ist und zum Erzeugen von Methan geeignet ist; und
- Solarkollektoren (19) und ein Mittel zur Wärmeübertragung von den Solarkollektoren zur Kohlendioxid-Abscheidungsvorrichtung (16).

2. Anlage (10) nach Anspruch 1, die eine Vorrichtung zum Auffangen von Ablaufwasser aus den Solarkollektoren (19) umfasst, wobei der Elektrolyseur (14) einen Einlass für an den Solarkollektoren (19) aufgefangenes Ablaufwasser umfasst.

3. Anlage (10) nach einem der Ansprüche 1 oder 2, wobei die elektrische Energiequelle (11) Photovoltaikmodule umfasst, wobei die Anlage eine Vorrichtung zum Auffangen von Ablaufwasser aus den Photovoltaikmodulen (11) umfasst, wobei der Elektrolyseur (14) einen Einlass für an den Photovoltaikmodulen (11) aufgefangenes Ablaufwasser umfasst.

4. Anlage (10) nach einem der Ansprüche 1 bis 3, die eine Dehydrierungseinheit (20) für die Methanabgabe aus dem Methanisierungsreaktor (15) umfasst, wobei der Methanisierungsreaktor einen Einlass für Wasser umfasst, das aus der Dehydrierungseinheit kommt.

5. Anlage (10) nach einem der Ansprüche 1 bis 4, die eine Dehydrierungseinheit (20) für die Methanabgabe aus dem Methanisierungsreaktor (15) umfasst, die für die Versorgung des Elektrolyseureinlasses (14) mit Wasser geeignet ist.

6. Anlage (10) nach einem der Ansprüche 1 bis 5, wobei der Elektrolyseur (14) zum Elektrolysieren der Wasserabgabe aus dem Methanisierungsreaktor (15) geeignet ist.

7. Anlage (10) nach einem der Ansprüche 1 bis 6, wobei der Elektrolyseur (14) zum Elektrolysieren der Wasserabgabe aus der Kohlendioxid-Abscheidungsvorrichtung aus der Atmosphäre (16) geeignet ist.

8. Anlage (10) nach einem der Ansprüche 1 bis 7, die ein Wärmeübertragungsmittel umfasst, das zum Übertragen von Wärme vom Elektrolyseur (14) auf die Kohlendioxid-Abscheidungsvorrichtung (16) geeignet ist.

9. Anlage (10) nach einem der Ansprüche 1 bis 8, die ein Wärmeübertragungsmittel umfasst, das zum Übertragen von Wärme vom Methanisierungsreaktor (15) auf die Kohlendioxid-Abscheidungsvorrichtung (16) geeignet ist.

10. Anlage (10) nach einem der Ansprüche 1 bis 9, wobei die elektrische Energiequelle Photovoltaikmodule (11) umfasst, die mit einem Kühlsystem ausgestattet sind, wobei die Anlage ein Wärmeübertragungsmittel umfasst, das zum Übertragen von Wärme von den Photovoltaikmodulen auf die Kohlendioxid-Abscheidungsvorrichtung (16) geeignet ist.

11. Anlage (10) nach einem der Ansprüche 1 bis 10, die einen Verdichter (17) zum Verdichten des vom Methanisierungsreaktor (15) erzeugten Methans und Einspritzen in ein Gastransport- oder -verteilungsnetz (18) umfasst; und ein Wärmeübertragungsmittel, das zum Übertragen von Wärme vom Verdichter auf die Kohlendioxid-Abscheidungsvorrichtung (16) geeignet ist.

12. Anlage (10) nach einem der Ansprüche 1 bis 11, wobei der Methanisierungsreaktor (15) ein Methanogenesereaktor ist.

13. Anlage (10) nach einem der Ansprüche 1 bis 11, wobei der Methanisierungsreaktor (15) ein katalytischer Methanisierungsreaktor ist.

14. Anlage (10) nach einem der Ansprüche 1 bis 11, wobei der Methanisierungsreaktor (15) ein thermochemischer Methanogenesereaktor ist.

## Claims

1. Plant (10) for producing methane, **characterised in that** it comprises:
- an electric energy source (11);
- a water electrolyser (14) supplied with electrical energy from the electric energy source, suitable for producing hydrogen in gas form;
- an atmospheric carbon dioxide capture device (16) suitable for producing carbon dioxide and water;
- a methanation reactor (15) fitted with an inlet for hydrogen from the electrolyser, fitted with an inlet for water and carbon dioxide from the atmospheric carbon dioxide capture device, and suitable for producing methane; and
- solar collectors (19) and a means for heat transfer from the solar collectors to the carbon dioxide capture device (16).

2. Plant (10) according to claim 1, which comprises a means of collecting run-off water from the solar collectors (19), the electrolyser (14) comprising an inlet for run-off water collected on the solar collectors (19).

3. Plant (10) according to one of claims 1 or 2, wherein the electrical energy source (11) comprises photovoltaic panels, the plant comprising a means of collecting run-off water from the photovoltaic panels (11), the electrolyser (14) comprising an inlet for run-off water collected on the photovoltaic panels (11).

4. Plant (10) according to one of claims 1 to 3, which comprises a dehydration unit (20) for the methane output by the methanation reactor (15), the methanation reactor comprising an inlet for water coming from the dehydration unit.

5. Plant (10) according to one of claims 1 to 4, which comprises a dehydration unit (20) for the methane output by the methanation reactor (15), suitable for supplying water to the electrolyser inlet (14).

6. Plant (10) according to one of claims 1 to 5, wherein the electrolyser (14) is suitable for electrolysing water output by the methanation reactor (15).

7. Plant (10) according to one of claims 1 to 6, wherein the electrolyser (14) is suitable for electrolysing water output from the atmospheric carbon dioxide capture device (16).

8. Plant (10) according to one of claims 1 to 7, which comprises a heat transfer means suitable for transferring heat from the electrolyser (14) to the carbon dioxide capture device (16).

9. Plant (10) according to one of claims 1 to 8, which comprises a heat transfer means suitable for transferring heat from the methanation reactor (15) to the carbon dioxide capture device (16).

10. Plant (10) according to one of claims 1 to 9, wherein the electrical energy source comprises photovoltaic panels (11) fitted with a cooling system, the plant comprising a heat transfer means suitable for transferring heat from the photovoltaic panels to the carbon dioxide capture device (16).

11. Plant (10) according to one of claims 1 to 10, which comprises a compressor (17) for compressing the methane produced by the methanation reactor (15) and injecting it into a gas transportation or distribution grid (18); and a heat transfer means suitable for transferring heat from the compressor to the carbon dioxide capture device (16).

12. Plant (10) according to one of claims 1 to 11, wherein the methanation reactor (15) is a methanogenesis reactor.

13. Plant (10) according to one of claims 1 to 11, wherein the methanation reactor (15) is a catalytic methanation reactor.

14. Plant (10) according to one of claims 1 to 11, wherein the methanation reactor (15) is a thermochemical methanogenesis reactor.
